# NEW EUROPEAN PATENT SPECIFICATION

(11) **EP 0 678 015 B2**
(45) Date of publication and mention of the opposition decision: **31.01.2001**
(45) Mention of the grant of the patent: 27.08.1997
(21) Application number: 94906059.4
(22) Date of filing: 10.01.1994
(51) Int. Cl.: A61K 7/42, A61K 7/00, A61K 7/021

(54) **COSMETIC COMPOSITIONS CONTAINING SURFACE TREATED PIGMENTS**
KOSMETISCHE MITTEL ENTHALTEND OBERFLÄCHIG BEHANDELTE PIGMENTE
COMPOSITIONS COSMETIQUES CONTENANT DES PIGMENTS TRAITES EN SURFACE

(30) Priority: 11.01.1993 US 3086
(43) Date of publication of application: 25.10.1995
(73) Proprietor: THE PROCTER & GAMBLE COMPANY, Cincinnati, Ohio 45202 (US)
(72) Inventor: JORGENSEN, Lise Wivestad, Woodbury, CT 06798 (US)
(74) Representative: Woof, Victoria
(86) International application number: US9400306
(87) International publication number: WO9415580

(56) References cited:
- EP-A- 0 193 387
- EP-A- 0 518 772
- WO-A-90/11067
- WO-A-93/04666
- WO-A-94/04131
- AU-A- 1 602 592
- US-A- 5 028 417
- Cosmetics & Toiletries, Vol. 105,February 1990, p. 53-64
- Cosmetics & Toiletries, vol. 105, December 1990, p. 92-93

## Description

The present invention relates to tinted oil-in-water emulsion compositions containing surface treated pigments. These compositions also contain a carboxylic acid polymer thicker, a chemical sunscreen, and a humectant. These compositions are useful for providing color, moisturization, and protection from the harmful effects of ultraviolet radiation, all from a single product.

### BACKGROUND OF THE INVENTION

Women have used cosmetic products to care for their skin since the dawn of civilization. These products have ranged from simple, commonly-available materials such as honey and plant extracts to hi-tech synthetic ingredients in a bewildering array of product forms.

Today, as part of their daily beauty regimen, many women use both a facial moisturizer and a foundation product. Typically, the moisturizer is first applied to the face followed by the foundation. Moisturizer products usually contain humectants and other ingredients to help keep the skin hydrated. Foundation products contain pigments for providing a uniform base color and to help hide imperfections, i.e. to provide a make-up benefit. In addition to moisturizers and foundations, many women also use a separate sunscreen product to protect the skin from the harmful effects of the sun's ultraviolet radiation.

The damaging effects of sunlight on skin are well documented and include erythema (i.e. sunburn), malignant changes in the skin surface, and premature aging of the skin. See DeSimone, "Sunscreen and Suntan Products", Handbook of Nonprescription Drugs, 7th Ed., Chapter 26, pp. 499-511 (American Pharmaceutical Association, Washington, D.C.; 1982); Grove and Forbes, "A Method for Evaluating the Photoprotection Action of Sunscreen Agents Against UV-A Radiation", International Journal of Cosmetic Science, 4, pp. 15-24 (1982); and U.S. Patent 4,387,089, DePolo, issued June 7, 1983; the disclosures of all of which are incorporated herein by reference.

Therefore, if an individual wants to moisturize one's skin, obtain color, and be protected against UV radiation, it is usually necessary to apply three separate products. Also, the use of three separate products might not be desirable, because each product can interfere with the performance of the others. For example, the pigments in a foundation product can disrupt the film formation of a suscreen product, thereby diminishing its effectiveness. Therefore, in order to provide convenience and insure efficacy, it would be highly desirable to compatibly deliver all three benefits from a single product.

Formulating a single product to provide color, moisturization, and UV protection benefits, however, is not as simple as combining all the desired ingredients. For example, it is known that foundation pigments (e.g., iron oxides) are not compatible with carboxylic acid polymer thickeners, which are often used to provide light, non-greasy, oil-in-water bases for moisturizers and sunscreens. Furthermore, the pigments can also interfere with the performance of the chemical sunscreen agents.

The present invention overcomes these compatibility issues by employing surface treated pigments. Surface treated pigments are known in the cosmetic industry, however, their use heretofore has been primarily limited to anhydrous-based products such as lipsticks, powders, mascaras, and to heavy, oil-based emulsions. It has been found in the present invention that surface treated pigments are compatible with carboxylic acid polymer thickeners and with chemical sunscreen agents. These treated pigments thus provide the ideal solution for preparing oil-in-water emulsions which deliver color, moisturization, and UV protection from a single product. These pigments are also be easier to incorporate into the oil phase of an emulsion for providing better distribution upon the skin and a more even and uniform color. The resulting emulsion products are light and non-greasy and have a smooth, silky, feel upon the skin.

It is therefore an object of the present invention to provide oil-in-water cosmetic emulsions which provide color, moisturization, and protection from the harmful effects of ultraviolet radiation from a single product.

It is another object of the present invention to provide emulsions containing a surface treated pigment, a carboxylic acid polymer thickener, a humectant, and a chemical sunscreen in a single product.

It is a further object of the present invention to provide methods for providing color, moisturization, and protection against the harmful effects of UV radiation by applying to the skin a single oil-in-water emulsion product.

These and other objects will become readily apparent from the detailed description which follows.

### SUMMARY OF THE INVENTION

The present invention relates to a tinted oil-in-water emulsion composition, useful for providing color, moisturization, and protection from the harmful effects of UV radiation, comprising:
(a) from about 0.1% to about 20% of finely divided pigment particles which have been surface treated as defined in claim 1 to make the particles more hydrophobic,
(b) from about 0.025% to about 1% of a carboxylic acid polymer thickener,
(c) from about 0.1% to about 30% of a chemical sunscreen agent,
(d) from about 0.1% to about 20% by weight of a humectant, and
(e) a cosmetically acceptable carrier.

All percentages and ratios used herein are by weight of the total composition and all measurements made are at 25°C, unless otherwise designated.

### DETAILED DESCRIPTION OF THE INVENTION

The term "tinted oil-in-water emulsion" as used herein is intended to include emulsion compositions which are tinted by the addition of surface treated pigments, wherein these pigments can be white, black, or grey, in addition to the primary colors, the secondary colors, and combinations thereof.

The term "for providing color" as used herein includes modifying the appearance of the skin by the application to the skin of a tinted emulsion composition.

The term "make-up benefit" as used herein includes modifying the appearance of the skin to provide a cosmetic benefit by applying a composition which provides a more uniform skin tone or color or which helps to hide or mask imperfections of the skin.

The compositions of the present invention comprise the following essential as well as optional components.

### Essential Components

### Surface Treated Pigments

The compositions of the present invention comprise from about 0.1% to about 20%, more preferably from about 1% to about 15% and most preferably from about 5% to about 10% of finely divided pigment particles which have been surface treated as defined hereinafter to make their surfaces more hydrophobic. Mixtures of more than one type of surface treated pigment can be used in the compositions described herein in order to achieve a wider variety of colors than can be obtained from a single treated pigment.

In general, pigments are small, discrete, solid particles used for opacifying, decorative, or protective purposes. Pigments are usually insoluble in the medium in which they are employed and have found widespread use in the cosmetics industry. See Hawley's Condensed Chemical Dictionary, Eleventh Edition, p. 919 (Van Nostrand Reinhold Co., New York 1987); and The Encyclopedia of Chemistry, Third Edition, pp. 859-861 (Van Nostrand Reinhold Co., New York: 1973), both of which are incorporated by reference herein.

Without being limited by theory, pigments are generally hydrophilic and possess relatively high surface activity. These properties can make it difficult to formulate a pigment into the oil phase of a water-in-oil emulsion. Furthermore, the high surface activity of a pigment can cause it to react with or interact with other ingredients commonly found in a cosmetic emulsion, e.g., carboxylic acid polymer thickeners and sunscreens. These disadvantages of pigments can be overcome by treating the surface of the pigment particle with a material which renders the surface more hydrophobic (i.e. less hydrophilic). In other words, the term "pigment particles which have been surface treated", as used herein, means that the surface of the pigment particle has been treated with a material to make the surface more hydrophobic. Additionally, it is also believed that these surface treatments can make the surface of the pigment particles less reactive so that the pigment particles are less likely to react with or catalyze reactions of other components commonly used in cosmetic emulsions.

Furthermore, the surface treated pigments used herein have the advantage of being easier to formulate into the oil phase of an oil-in-water emulsion. Additionally, when these emulsions are applied to the skin they provide a more uniform pigment distribution and a smoother, silkier feel, compared to untreated pigments.

A wide variety of pigments can be surface treated for use in the present invention. These pigments include those selected from the group consisting of iron oxide, titanium dioxide, zinc oxide, bismuth oxychloride, calcium silicate, chromium oxide, chromium hydroxide, ferric ammonium ferrocyanide, ferric ferrocyanide, kaolin, manganese violet, mica, silica, talc, ultramarine, and mixtures thereof. More preferred for use in the present invention are those pigments selected from the group consisting of iron oxide, talc, titanium dioxide, zinc oxide, and mixtures thereof. Most preferred for use in the present invention are those pigments selected from the group consisting of iron oxide, talc, titanium dioxide, and mixtures thereof.

As used herein the iron oxides are intended to include the various forms and color varieties of iron oxide, non-limiting examples of which include iron oxides selected from the group consisting of black iron oxide, brown iron oxide, orange iron oxide, red iron oxide, russet iron oxide, umber iron oxide, yellow iron oxide, and mixtures thereof.

The pigment particles used herein can span a broad range of colors including white, black, and grey, in addition to the primary colors, the secondary colors, and combinations thereof. Mixtures of different colored pigment particles can be used to achieve subtle gradations of color. Also, the pigment particles of the present invention are "finely divided", which means that the particles have been reduced to a small enough size for ready and uniform dispersion into an emulsion composition without individual particles being visible to the naked eye or giving a gritty feel. Preferably the finely divided pigment particles should have an average particle size below about 600 nm, more preferably the average particle size should be from about 30 nm to about 600 nm, and most preferably from about 30 nm to about 300 nm.

The surface treatment materials used to surface treat the pigment particles of the present invention can comprise from about 0.1% to about 50%, more preferably from about 0.25% to about 25%, and most preferably from about 0.5% to about 10% by weight of the surface treated pigment.

The surface treatment materials useful for treating the pigment particles include N-acyl amino acids, and mixtures thereof.

Examples of N-acyl amino acids useful as surface treatment materials include any of the amino acids, their N-methyl derivatives, or salts thereof, wherein the amino group has been acylated with a moiety derived from a C₁₀₋₅₀ straight or branched chain fatty acid.

Preferred salts are those selected from the group consisting of aluminum, magnesium, calcium, zinc, zirconium, titanium, and mixtures thereof, with aluminum being most preferred.

The surface treatment material for the pigment particles of the present invention is an N-acyl amino acid. The amino acid portion of these N-acyl amino acids can be derived from any of the naturally occurring amino acids with those derived from glycine, alanine, and glutamic acid being preferred. Even more preferred is when the N-acyl amino acid is derived from an N-methyl amino acid, with N-methyl glycine, N-methyl alanine, and N-methyl glutamic acid being most preferred. In any of these N-acyl amino acids, the acyl group is preferably derived from a C₁₀₋₅₀ straight or branched chain fatty acid, more preferably from a C₁₀₋₃₀ straight or branched chain fatty acid, and most preferably from a C₁₀₋₂₀ straight or branched chain fatty acid. Nonlimiting examples of useful acyl groups include those acyl groups derived from capric acid, lauric acid, myristic acid, palmitic acid, stearic acid, oleic acid and mixtures thereof. It has been found herein that salts of the N-acyl amino acids are especially useful surface treatment materials. Preferred salts are those selected from the group consisting of aluminum, magnesium, calcium, zinc, zirconium, titanium, and mixtures thereof, with aluminum being most preferred.

The N-acyl amino acid metal salt surface treated pigment particles can be manufactured in the following manner. First, a pigment material (including any of which have been described above, or alternatively a pigment extender as described in the optional components below) is suspended in water to form about a 5% to about a 30% by weight suspension. To this suspension is added an N-acyl amino acid water-soluble salt (e.g., a sodium, potassium, aluminum, magnesium, calcium, zinc, zirconium, or titanium salt can be employed, with sodium being preferred) in an amount of 0.5% to 10%, preferably from about 1% to about 4%, by weight with respect to the pigment and stirring is carried out to form a homogeneous suspension. To this suspension is added about 1% to about 30%, preferably from about 5% to about 10%, by weight of a water soluble salt of aluminum, magnesium, calcium, zinc, zirconium, or titanium. Nonlimiting examples of suitable water-soluble salts include aluminum nitrate, aluminum chloride, aluminum sulfate, potassium aluminum sulfate, magnesium chloride, magnesium sulfate, magnesium nitrate, potassium magnesium sulfate, calcium chloride, calcium nitrate, calcium acetate, zinc chloride, zinc nitrate, zinc sulfate, zinc acetate, zirconium sulfate, zirconium chloride, titanium oxysulfate, titanium tetrachloride and the like. These salts are employed in amounts such that the amount of the water-soluble salt of the metal is about 0.65 to about 2 molar equivalents, preferably about 1 to about 1.2 molar equivalents, with respect to the N-acyl amino acid water-soluble salt. Without being limited by theory, it is thereby believed that the N-acyl amino acid water-soluble salt reacts with the water-soluble metal salt to cause the N-acyl amino acid metal salt to be deposited onto the surface of the pigment. The process is completed by stirring for about 10 minutes and then concentrating by any suitable means including centrifuging, rotary evaporation, freeze drying, and the like to yield the surface treated pigment. Nonlimiting examples of N-acyl amino acid metal treatment materials, the resulting surface treated pigments, and methods for making the these are disclosed in U.S. Patent No. 4,606,914, to Miyoshi, issued August 16, 1986, which is incorporated by reference herein in its entirety.

Nonlimiting examples of other methods of preparation are described in the following patents, which are all incorporated by reference herein in their entirety: U.S. Patent No. 5,143,722, to Hollenberg et al., issued September 1, 1992; U.S. Patent No. 4,919,922, to Miyoshi et al, issued April 24, 1990; U.S. Patent No. 4,863,800, to Miyoshi et al., issued September 5, 1989; French Patent No. 2,610,943, to Myoshi et al., published August 19, 1988; Japanese Patent No. 2,242,998, assigned to Asada Seifun KK et al., published September 27, 1990; Japanese Patent No. 62,087,237, assigned to Asada Seifun KK et al., published April 21, 1987; Japanese Patent No. 2,088,453, assigned to Shin Nippon Kagaku, published March 28, 1990; Japanese Patent No. 1,215,865, assigned to Miyoshi Kasei YG, published August 29, 1989; Japanese Patent No. 60,081,012, assigned to Miyoshi Kasei YG, published May 9, 1985; Japanes Patent No.63,215,616, assigned to Miyoshi Kasei KK et al., published September 8, 1988; and Japanese Patent No. 3024008, assigned to Kanebo Ltd., published February 1, 1991.

A variety of surface treated pigments useful herein are commercially available. For example Miyoshi Kasei of Japan sells various surface treated pigments through U.S. Cosmetics Corporation (Dayville, CT). Examples of these surface treated pigments include amino acid treated pigments (e.g., aluminum N-acyl glutamate treated), wherein the pigment particle can include iron oxide, talc, mica, titanium dioxide, and the like.

### Carobxylic Acid Polymer Thickener

The compositions of the present invention comprise a crosslinked carboxylic acid polymer thickener. These crosslinked polymers contain one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol. The preferred polymers for use herein are of two general types. The first type of polymer is a crosslinked homopolymer of an acrylic acid monomer or derivative thereof (e.g., wherein the acrylic acid has substituents on the two and three carbon positions independently selected from the group consisting of C₁₋₄ alkyl, -CN, -COOH, and mixtures thereof). The second type of polymer is a crosslinked copolymer having a first monomer selected from the group consisting of an acrylic acid monomer or derivative thereof (as just described in the previous sentence), a short chain alcohol (i.e. a C₁₋₄) acrylate ester monomer or derivative thereof (e.g., wherein the acrylic acid portion of the ester has substituents on the two and three carbon positions independently selected from the group consisting of C₁₋₄ alkyl, -CN, -COOH, and mixtures thereof), and mixtures thereof; and a second monomer which is a long chain alcohol (i.e. C₈₋₄₀) acrylate ester monomer or derivative thereof (e.g., wherein the acrylic acid portion of the ester has substituents on the two and three carbon positions independently selected from the group consisting of C₁₋₄ alkyl, -CN, -COOH, and mixtures thereof). Combinations of these two types of polymers are also useful herein.

In the first type of crosslinked homopolymers the monomers are preferably selected from the group consisting of acrylic acid, methacrylic acid, ethacrylic acid, and mixtures thereof, with acrylic acid being most preferred. In the second type of crosslinked copolymers the acrylic acid monomer or derivative thereof is preferably selected from the group consisting of acrylic acid, methacrylic acid, ethacrylic acid, and mixtures thereof, with acrylic acid, methacrylic acid. and mixtures thereof being most preferred. The short chain alcohol acrylate ester monomer or derivative thereof is preferably selected from the group consisting of C₁₋₄ alcohol acrylate esters, C₁₋₄ alcohol methacrylate esters, C₁₋₄ alcohol ethacrylate esters, and mixtures thereof, with the C₁₋₄ alcohol acrylate esters, C₁₋₄ alcohol methacrylate esters, and mixtures thereof, being most preferred. The long chain alcohol acrylate ester monomer is selected from C₈₋₄₀ alkyl acrylate esters, with C₁₀₋₃₀ alkyl acrylate esters being preferred.

The crosslinking agent in both of these types of polymers is a polyalkenyl polyether of a polyhydric alcohol containing more than one alkenyl ether group per molecule, wherein the parent polyhydric alcohol contains at least 3 carbon atoms and at least 3 hydroxyl groups. Preferred crosslinkers are those selected from the group consisting of allyl ethers of sucrose and allyl ethers of pentaerythritol, and mixtures thereof. These polymers useful in the present invention are more fully described in U.S. Patent No. 5,087,445, to Haffey et al., issued February 11, 1992; U.S. Patent No. 4,509,949, to Huang et al., issued April 5, 1985; U.S. Patent No. 2,798,053, to Brown, issued July 2, 1957; which are incorporated by reference herein. See also, CTFA International Cosmetic Ingredient Dictionary, fourth edition, 1991, pp. 12 and 80; which are also incorporated herein by reference.

Examples of commercially availble hompolymers of the first type useful herein indude the carbomers, which are homopolymers of acrylic acid crosslinked with allyl ethers of sucrose or pentaerytritol. The carbomers are available as the Carbopol^{R} 900 series from B.F. Goodrich. Examples of commercially available copolymers of the second type useful herein include copolymers of C₁₀₋₃₀ alkyl acrylates with one or more monomers of acrylic acid, methacrylic acid, or one of their short chain (i.e. C₁₋₄ alcohol) esters, wherein the crosslinking agent is an allyl ether of sucrose or pentaerytritol. These copolymers are known as acrylates/C10-30 alkyl acrylate crosspolymers and are commerically available as Carbopol^{R} 1342, Pemulen TR-1, and Pemulen TR-2, from B.F Goodrich. In other words, examples of carboxylic acid polymer thickeners useful herein are those selected from the group consisting of carbomers, acrylates/C10-C30 alkyl acrylate crosspolymers, and mixtures thereof.

The compositions of the present invention comprise from about 0.025% to about 1%, more preferably from about 0.05% to about 0.75% and most preferably from about 0.10% to about 0.50% of the carboxylic acid polymer thickeners.

### Chemical Sunscreen Agents

The compositions of the present invention comprise at least one chemical sunscreen agent. By chemical sunscreen agent is meant a sunscreen that is an organic compound or salt thereof as opposed to an inorganic particulate material. These chemical sunscreen agents are soluble in the water or oil phase of the emulsion and absorb UV radiation, in contrast to particulate inorganic sunscreens. A wide variety of one or more chemical sunscreen agents are suitable for use in the present invention and are described in U.S. Patent No. 5,087,445, to Haffey et al., issued February 11, 1992; U.S. Patent No. 5,073,372, to Turner et al., issued December 17, 1991; U.S. Patent No. 5,073,371, to Turner et al. issued December 17, 1991; Segarin, et al., at Chapter VIII, pages 189 et seq., of Cosmetics Science and Technology; U.S. Patent No. 4,937,370, to Sabatelli, issued June 26, 1990; and U.S. Patent No. 4,999,186, to Sabatelli et al., issued March 12, 1991; all of which are incorporated herein by reference in their entirety.

Preferred among those chemical sunscreen agents which are useful in the compositions of the instant invention are those selected from the group consisting of ethylhexyl p-methoxycinnamate, octyl salicylate, octocrylene, oxybenzone, 2-ethylhexyl N,N-dimethylaminobenzoate, 2-phenyl-benzimidazole-5-sulfonic acid, homomenthyl salicylate, 4,4'methoxy-t-butyldibenzoylmethane, 4-isopropyldibenzoylmethane, 3-(4-methylbenzylidene) camphor, 3-methylbenzylidene camphor, 4-N,N-dimethylaminobenzoic acid ester with 2,4-dihydroxybenzophenone, 4-N,N-dimethylaminobenzoic acid ester with 2-hydroxy-4-(2-hydroxyethoxy)benzophenone, 4-N,N-dimethylaminobenzoic acid ester with 4-hydroxydibenzoylmethane, 4-N,N-dimethylaminobenzoic acid ester with 4-(2-hydroxyethoxy)dibenzoylmethane, 4-N,N-di(2-ethylhexyl)-aminobenzoic acid ester with 2,4-dihydroxybenzophenone, 4-N,N-di(2-ethylhexyl)aminobenzoic acid ester with 2-hydroxy-4- (2-hydroxyethoxy)benzophenone, 4-N,N-di(2-ethylhexyl)aminobenzoic acid ester with 4-hydroxydibenzoylmethane, 4-N,N-di(2-ethylhexyl)aminobenzoic acid ester with 4-(2-hydroxyethoxy)dibenzoylmethane, 4-N,N-(2-ethylhexyl)methylaminobenzoic acid ester with 2,4-dihydroxybenzophenone, 4-N,N-(2-ethylhexyl )methylaminobenzoic acid ester with 2-hydroxy-4-(2-hydroxyethoxy)benzophenone, 4-N,N-(2-ethylhexyl)methylaminobenzoic acid ester with 4-hydroxydibenzoylmethane, 4-N,N-(2-ethylhexyl )methylaminobenzoic acid ester with 4-(2-hydroxyethoxy)dibenzoylmethane, and mixtures thereof. More preferred for use in the compositions described herein are the sunscreen agents selected from the group consisting of ethylhexyl p-methoxycinnamate, octocrylene, octyl salicylate, oxybenzone, 2-phenylbenzimldazole-5-sulfonic acid, 4,4'-methoxy-t-buyl-methoxydibenzoylmethane, 3-(4-methylbenzylidene) camphor, and mixtures thereof.

The chemical sunscreen agents can comprise from about 0.1% to about 30%, preferably from about 0.5% to about 20%, more preferably from about 0.75% to about 15%, and most prefereably from about 1% to about 10% of the compositions of the present invention. Exact amounts will vary depending upon the sunscreen chosen and the desired Sun Protection Factor (SPF). SPF is a commonly used measure of photoprotection of a sunscreen against erythema. The SPF is defined as the ratio of the ultraviolet energy required to produce minimal erythema on protected skin to that required to produce the same minimal erythema on unprotected skin in the same individual. See Federal Register, Vol. 43. No. 166, pp. 38206-38269, August 25, 1978, which is incorporated herein by reference in its entirety.

### Humectants

The compositions of the present invention comprise at least one humectant. As is well known in the art, a humectant is a material that promotes retention of moisture and which is useful for moisturizing the skin. Even though these materials are defined herein as humectants, they can also possess moisturizing, skin conditioning, and other related properties. A variety of humectants can be employed and can be present at a level of from about 0.1% to about 20%, more preferably from about 0.5% to about 10%, and most preferably from about 1 % to about 5%.

Examples of humectants useful herein include materials such as urea; guanidine; glycolic acid and glycolate salts (e.g. ammonium and quaternary alkyl ammonium); lactic acid and lactate salts (e.g. ammonium and quaternary alkyl ammonium); aloe vera in any of its variety of forms (e.g., aloe vera gel); polyhydroxy alcohols such as sorbitol, glycerol, hexanetriol, propylene glycol, butylene glycol, hexylene glycol, and the like; polyethylene glycol; sugars and starches; sugar and starch derivatives (e.g., alkoxylated glucose); hyaluronic acid; chitin, starch-grafted sodium polyacrylates such as Sanwet (RTM) IM-1000, IM-1500, and IM-2500 (available from Celanese Superabsorbent Materials, Portsmouth, VA); lactamide monoethanolamine; acetamide monoethanolamine; and mixtures thereof.

Preferred humectants useful in the compositions of the present invention are the C3-C6 diols and triols. More preferred as humectants are the C3-C6 diols and triols selected from the group consisting of propylene glycol, 1,3-dihydroxypropane, glycerin, butylene glycol, hexylene glycol, 1,4-dihydroxyhexane, 1,2,6-hexanetriol,and mixtures thereof. Most preferred as humectants are those selected from the group consisting of glycerin, butylene glycol, hexylene glycol, and mixtures thereof. Among these humectants, glycerin is especially preferred.

### Cosmetically Acceptable Carrier

The compositions of the present invention comprise as a necessary component a cosmetically acceptable carrier or diluent which can be of a variety of different forms. By "cosmetically acceptable" is meant that the carrier comprises common pharmaceutical and cosmetic ingredients which are typically used in the industry and which are generally recognized as safe for human contact. The topical carrier can be in the form of an emulsion including, but not limited to, oil-in-water, water-in-oil, water-in-oil-in-water, and oil-in-water-in-silicone emulsions. These emulsions can cover a broad range of consistencies including thin lotions (which can also be suitable for spray or aerosol delivery), creamy lotions, light creams, heavy creams, and the like. Examples of topical carrier systems useful in the present invention are described in the following four references all of which are incorporated herein by reference in their entirety: "Sun Products Formulary" Cosmetics & Toiletries, vol. 105, pp. 122-139 (December 1990); "Sun Products Formulary", Cosmetics & Toiletries, vol. 102, pp. 117-136 (March 1987); U.S. Patent No. 4,960,764 to Figueroa et al., issued October 2,1990; and U.S. Patent No. 4,254,105 to Fukuda et al., issued March 3, 1981.

The pharmaceutically-acceptable topical carriers, in total, typically comprise from about 29% to about 99.675% by weight of the sunscreen compositions of the present invention, preferably from about 60% to about 99%, and most preferably from about 70% to about 95%.

A preferred topical carrier of the compositions of the present invention is an oil-in-water type emulsion.

### Other Components

In addition to the essential components listed above, the compositions of the present invention can comprise the following additional components.

### Emulsifiers

Suitable emulsifiers can include any of a wide variety of nonionic, cationic, anionic, and zwitterionic emulsifiers disclosed in the prior patents and other references. See McCutcheon's, Detergents and Emulsifiers, North American Edition (1986), published by Allured Publishing Corporation; U.S. Patent No. 5,011,681 to Ciotti et al., issued April 30, 1991; U.S. Patent No. 4,421,769 to Dixon et al., issued December 20, 1983; and U.S. Patent No. 3,755,560 to Dickert et al., issued August 28, 1973; these four references are incorporated herein by reference in their entirety.

Suitable emulsifier types include esters of glycerin, esters of propylene glycol, fatty acid esters of polyethylene glycol, fatty acid esters of polypropylene glycol, esters of sorbitol, esters of sorbitan anhydrides, carboxylic acid copolymers, esters and ethers of glucose, ethoxylated ethers, ethoxylated alcohols, alkyl phosphates, polyoxyethylene fatty ether phosphates, fatty acid amides, acyl lactylates, soaps and mixtures thereof.

Suitable emulsifiers can include, but are not limited to, polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, PEG-100 stearate, and mixtures thereof.

The emulsifiers can be used individually or as a mixture of two or more and comprise from about 0.1% to about 10%, more preferably from about 1% to about 7%, and most preferably from about 1% to about 5% of the compositions of the present invention.

### Emollients

Examples of suitable emollients indude, but are not limited to, volatile and non-volatile silicone oils (e.g., dimethicone, cyclomethicone, dimethiconol, and the like), highly branched hydrocarbons, and non-polar carboxylic acid and alcohol esters, and mixtures thereof. Emollients useful in the instant invention are further described in U.S. Patent No. 4,919,934, to Deckner et al., issued April 24 1990, which is incorporated herein by reference in its entirety.

The emollients can typically comprise in total from about 0.5% to about 50%, more preferably from about 0.5% to about 25%, and most preferably from about 0.5% to about 10% by weight of the compositions useful in the present invention.

### Pigment Extenders

The compositions of the present invention can include various pigment extenders. Pigment extenders are not true pigments but are finely divided particulate materials which can be used in combination with pigments to facilitate the handling of the pigments and also to reduce or dilute the color intensity of the pigment particles. Examples of pigment extenders that can be used in combination with the surface treated pigments herein include magnesium carbonate, calcium carbonate, aluminum silicate, magnesium silicate, calcium silicate, clay, silica, and mixtures thereof. The pigment extenders, when employed herein, are also preferably surface treated in the same manner as described herein for the pigment particles.

### Additional Components

A variety of additional components can be incorporated into the compositions useful in the compositions of the present invention. Non-limiting examples of these additional ingredients include vitamins and derivatives thereof (e.g., ascorbic acid, vitamin E, tocopheryl acetate, retinoic acid, retinol, retinoids, and the like); thickening agents (e.g. polyacrylamide and C₁₃₋₁₄ isoparaffin and laureth-7, available as Sepigel from Seppic Corporation; polyquaternium and mineral oil, available as Salcare SC92, from Allied Colloids; crosslinked methyl quaternized dimethylaminomethacrylate and mineral oil, available as Salcare SC95 from Allied Colloids; resins; gums and thickeners such as xanthan gum, carboxymethyl cellulose, hydroxymethyl cellulose, hydroxyethyl cellulose, and magnesium aluminum silicate; cationic polymers and thickeners (e.g., cationic guar gum derivatives such as guar hydroxypropyltrimonium chloride and hydroxypropyl guar hydroxypropyltrimonium chloride, available as the Jaguar C series from Rhone-Poulenc; polymers for aiding the film-forming properties and substantivity of the composition (such as a copolymer of eicosene and vinyl pyrrolidone, an example of which is available from GAF Chemical Corporation as Ganex^{R} V-220); preservatives for maintaining the antimicrobial integrity of the compositions; skin penetration aids such as DMSO, 1-dodecylazacycloheptan-2-one (available as Azone from the Upjohn Co.) and the like; anti-acne medicaments (resorcinol, salicylic acid, erythromycin, benzoyl peroxide, zinc, and the like); artificial tanning agents such as dihydroxyacetone and the like; skin bleaching (or lightening) agents including but not limited to hydroquinone, kojic acid and sodium metabisulfite; antioxidants; chelators and sequestrants; and aesthetic components such as fragrances, colorings, essential oils, skin sensates, astringents, skin soothing agents, skin healing agents and the like, nonlimiting examples of these aesthetic components include panthenol and derivatives (e.g. ethyl panthenol), pantothenic acid and its derivatives, clove oil, menthol, camphor, eucalyptus oil, eugenol, menthyl lactate, witch hazel distillate, allantoin, bisabalol, dipotassium glycyrrhizinate and the like.

Among these additional components, those that are especially preferred include the artificial tanning agent dihydroxyacetone, and thickeners such as xanthan gum, carboxymethyl cellulose, hydroxymethyl cellulose, hydroxyethyl cellulose, and mixtures thereof. When used in these compositions, the dihydroxyacetone comprises from about 0.1% to about 10%, more preferably from about 0.5% to about 5%, and most preferably from about 1% to about 4%. When used in these compositions, the thickeners such as xanthan gum, carboxymethyl cellulose, hydroxymethyl cellulose, hydroxyethyl cellulose, magnesium aluminum silicate and mixtures thereof, comprise from about 0.05% to about 10%, more preferably from about 0.1% to about 5%, and most preferably from about 0.1% to about 1%.

### Methods

The compositions of the present invention are useful for providing a make-up benefit, moisturization, and protection against the harmful effects of UV radiation. Typically, these compositions are applied to the skin in an effective amount which is about 2 mg/cm².

### EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention.

Ingredients are identified by chemical or CTFA name.

### EXAMPLE I

A tinted cosmetic emulsion composition is prepared from the following components utilizing conventional mixing techniques.

### Cosmetic Emulsion

| Ingredients | % Weight |
|---|---|
| Water | QS 100 |
| Octyl Methoxycinnamate | 7.50 |
| 2-Phenylbenzimidazole 5-Sulfonic Acid | 1.00 |
| Isohexadecane | 3.00 |
| Butylene glycol | 2.00 |
| Triethanolamine | 1.02 |
| Glycerin | 3.00 |
| Stearic Acid | 1.00 |
| Cetyl Alcohol | 1.00 |
| DEA Cetyl Phosphate | 0.75 |
| Aluminum Starch Octenyl Succinate | 0.50 |
| Cetyl Palmitate | 0.50 |
| Dimethicone | 0.50 |
| Stearoxytrimethylsilane (and) Stearyl Alcohol | 0.50 |
| Imidazolidinyl Urea | 0.30 |
| Magnesium Aluminum Silicate | 0.30 |
| Xanthan Gum | 0.30 |
| Methylparaben | 0.25 |
| Propylparaben | 0.20 |
| Carbomer 954 | 0.10 |
| PEG-10Soya Sterol | 0.05 |
| Disodium EDTA | 0.05 |
| Castor Oil | 0.05 |
| Aluminum Acyl Glutamate Treated Talc J68¹ | 3.96 |
| Aluminum Acyl Glutamate Treated Titanium Dioxide 47501¹ | 3.00 |
| Aluminum Acyl Glutamate Treated Yellow Iron Oxide 338073¹ | 0.216 |
| Aluminum Acyl Glutamate Treated Russet Iron Oxide 338075¹ | 0.168 |
| Aluminum Acyl Glutamate Treated Russet Iron Oxide 335138¹ | 0.084 |
| Aluminum Acyl Glutamate Treated Black Iron Oxide 335198¹ | 0.048 |

| | |
|---|---|
| ¹ From Miyoshi Kasei of Japan via U.S. Cosmetics Corporation (Dayville. CT). | |

The above ingredients are combined to form an oil-in-water emulsion.

This composition is useful for topical application to the skin, especially the face, to provide color, moisturization, and protection from the harmful effects of ultraviolet radiation.

### EXAMPLE II

### Cosmetic Emulsion

A tinted cosmetic emulsion composition is prepared from the following components utilizing conventional mixing techniques.

| Ingredients | % Weight |
|---|---|
| Water | QS 100 |
| Octyl Methoxycinnamate | 7.50 |
| 2-Phenylbenzimidazole 5-Sulfonic Acid | 1.00 |
| Glycerin | 3.00 |
| Butylene glycol | 2.00 |
| Isohexadecane | 3.00 |
| Methylparaben | 0.25 |
| Magnesium Aluminum Silicate | 0.25 |
| Xanthan Gum | 0.20 |
| Disodium EDTA | 0.05 |
| Carbomer 954 | 0.20 |
| Dimethicone | 0.60 |
| Stearoxytrimethylsilane (and) Stearyl Alcohol | 0.50 |
| PEG-10 Soya Sterol | 0.10 |
| Stearic Acid | 1.00 |
| Cetyl Alcohol | 1.00 |
| Cetyl Palmitate | 0.50 |
| DEA Cetyl Phosphate | 0.75 |
| Aluminum Starch Octenyl Succinate | 0.50 |
| Propylparaben | 0.15 |
| Quaternium-22 | 1.00 |
| Dimethiconol (and) Dimethicone | 0.50 |
| Imidazolidinyl Urea | 0.30 |
| Triethanolamine | 1.02 |
| Aluminum Acyl Glutamate Treated Talc J68¹ | 3.96 |
| Aluminum Acyl Glutamate Treated Titanium Dioxide 47501¹ | 3.00 |
| Aluminum Acyl Glutamate Treated Yellow Iron Oxide 338073¹ | 0.216 |
| Aluminum Acyl Glutamate Treated Russet Iron Oxide 338075¹ | 0.168 |
| Aluminum Acyl Glutamate Treated Russet Iron Oxide 335138¹ | 0.084 |
| Aluminum Acyl Glutamate Treated Black Iron Oxide 335198¹ | 0.048 |

| | |
|---|---|
| ¹ From Miyoshi Kasei of Japan via U.S. Cosmetics Corporation (Dayville, CT). | |

The above ingredients are combined to form an oil-in-water emulsion.

This composition is useful for topical application to the skin, especially the face, to provide color, moisturization, and protection from the harmful effects of ultraviolet radiation.

Alternatively, Examples I and II are prepared in which the following levels of surface treated pigments are used:

| | |
|---|---|
| Aluminum Acyl Glutamate Treated Talc J68¹ | 4.165 |
| Aluminum Acyl Glutamate Treated Titanium Dioxide 47501¹ | 3.00 |
| Aluminum Acyl Glutamate Treated Yellow Iron Oxide 338073¹ | 0.140 |
| Aluminum Acyl Glutamate Treated Russet Iron Oxide 338075¹ | 0.109 |
| Aluminum Acyl Glutamate Treated Russet Iron Oxide 335138¹ | 0.055 |
| Aluminum Acyl Glutamate Treated Black Iron Oxide 335198¹ | 0.031 |

| | |
|---|---|
| ¹ From Miyoshi Kasei of Japan via U.S. Cosmetics Corporation (Dayville CT). | |

In another alternative, Examples I and II are prepared in which the following levels of surface treated pigments are used:

| | |
|---|---|
| Aluminum Acyl Glutamate Treated Talc J68¹ | 3.44 |
| Aluminum Acyl Glutamate Treated Titanium Dioxide 47501¹ | 3.00 |
| Aluminum Acyl Glutamate Treated Yellow Iron Oxide 338073¹ | 0.514 |
| Aluminum Acyl Glutamate Treated Russet Iron Oxide 338075¹ | 0.161 |
| Aluminum Acyl Glutamate Treated Russet Iron Oxide 335138¹ | 0.246 |
| Aluminum Acyl Glutamate Treated Black Iron Oxide 335198¹ | 0.139 |

| | |
|---|---|
| ¹ From Miyoshi Kasei of Japan via U.S. Cosmetics Corporation (Dayville, CT). | |

## Claims

1. A tinted oil-in-water emulsion composition, useful for providing color, moisturization, and protection from the harmful effects of UV radiation, comprising:
(a) from 0.1% to 20% of finely divided pigment particles which have been surface treated to make the particles more hydrophobic, with a material selected from N-acyl amino acids and salts thereof and mixtures thereof;
(b) from 0.025% to 1% of a carboxylic acid polymer thickener;
(c) from 0.1% to 30% of a chemical sunscreen agent;
(d) from 0.1% to 20% by weight of a humectant, and
(e) a cosmetically acceptable carrier.

2. A composition according to claim 1 wherein said pigment particles comprise from 5% to 10% of said composition and said pigment particles are selected from the group consisting of iron oxide, titanium dioxide, zinc oxide, bismuth oxychloride, calcium silicate, chromium oxide, chromium hydroxide, ferric ammonium ferrocyanide, ferric ferrocyanide, kaolin, manganese violet, mica, silica, talc, ultramarine, and mixtures thereof; preferably wherein said pigment particles are selected from the group consisting of iron oxide, talc, titanium dioxide, and mixtures thereof.

3. A composition according to claim 2 wherein said iron oxide pigment particles are selected from the group consisting of black iron oxide, brown iron oxide, orange iron oxide, red iron oxide, russet iron oxide, umber iron oxide, yellow iron oxide, and mixtures thereof.

4. A composition according to any of claims 1 to 3 wherein said N-acyl amino acid or salt thereof is selected from the group consisting of N-acyl N-methylglycine, N-acyl N-methylalanine, N-acyl N-methylglutamic acid, and mixtures thereof, and said acyl group is derived from a C₁₀₋₅₀ straight or branched chain fatty acid, preferably wherein said acyl group is derived from a fatty acid selected from the group consisting of capric acid, lauric acid, myristic acid, palmitic acid, stearic acid, oleic acid and mixtures thereof.

5. A composition according to claim 4 wherein said salts of said N-acyl amino acids are selected from the group consisting of aluminum, magnesium, calcium, zinc, zirconium, titanium, and mixtures thereof; preferably wherein said salt is an aluminum salt.

6. A composition according to claim 5 wherein said carboxylic acid polymer thickener comprises from 0.10% to 0.50% and is selected from the group consisting of carbomers, acrylates/C10-C30 alkyl acrylate crosspolymers, and mixtures thereof; said chemical sunscreen agent comprises from 1% to 10% and is selected from the group consisting of ethylhexyl p-methoxycinnamate, octocrylene, octyl salicylate, oxybenzone, 2-phenylbenzimidazole-5-sulfonic acid, 4,4'-methoxy-t-buyldibenzoylmethane, 3-(4-methylbenzylidene) camphor, and mixtures thereof; and said humectant comprises from 1% to 5% of said composition and is selected from the group consisting of glycerin, butylene glycol, hexylene glycol, and mixtures thereof; preferably wherein said humectant is glycerin.

7. A composition according to claim 6 wherein said composition further comprises from 0.1% to 1% of an additional thickener selected from the group consisting of xanthan gum, carboxymethyl cellulose, hydroxymethyl cellulose, hydroxyethyl cellulose, magnesium alumimum silicate and mixtures thereof.

8. A composition according to claim 7 wherein said composition further comprises from 1% to 4% of dihydroxyacetone.

9. The use of a composition in the manufacture of a medicament for providing color, moisturization, and protection against the harmful effects of UV radiation, said composition comprising:
(a) from 0.1% to 20% of finely divided pigment particles which have been surface treated to make the particles more hydrophobic, with a material selected from N-acyl amino acids and salts thereof and mixtures thereof;
(b) from 0.025% to 1% of a carboxylic acid polymer thickener;
(c) from 0.1% to 30% of a chemical sunscreen agent;
(d) from 0.1% to 20% by weight of a humectant, and
(e) a cosmetically acceptable carrier.

## Patentansprüche

1. Gefärbte Öl-in-Wasser-Emulsionszusammensetzung, welche zur Gewährleistung von Farbe, Befeuchtung und Schutz vor den schädlichen Wirkungen von UV-Strahlung nützlich ist, umfassend:
(a) 0,1 % bis 20 % von fein zerteilten Pigmentteilchen, welche mit einem unter N-Acylaminosäuren und Salzen hievon und Gemischen hievon ausgewählten Material oberflächenbehandelt wurden, um die Teilchen hydrophober zu machen,
(b) 0,025 % bis 1 % von einem Carbonsäurepolymer als Verdickungsmittel,
(c) 0,1 % bis 30 % von einem chemischen Sonnenschutzmittel,
(d) 0,1 Gew.-% bis 20 Gew.-% von einem Feuchthaltemittel und
(e) einen kosmetisch annehmbaren Träger.

2. Zusammensetzung nach Anspruch 1, worin die genannten Pigmentteilchen 5 % bis 10 % der genannten Zusammensetzung darstellen und die genannten Pigmentteilchen von der Gruppe ausgewählt sind, welche aus Eisenoxid, Titandioxid, Zinkoxid, Bismutoxychlorid, Calciumsilicat, Chromoxid, Chromhydroxid, Eisen(III)ammoniumferrocyanid, Eisen(III)-ferrocyanid, Kaolin, Manganviolett, Glimmer, Siliciumdioxid, Talk, Ultramarin und Gemischen hievon besteht; wobei die genannten Pigmentteilchen vorzugsweise von der Gruppe ausgewählt sind, welche aus Eisenoxid, Talk, Titandioxid und Gemischen hievon besteht.

3. Zusammensetzung nach Anspruch 2, wobei die genannten Eisenoxidpigmentteilchen von der aus schwarzem Eisenoxid, braunem Eisenoxid, orangem Eisenoxid, rotem Eisenoxid, rostbraunem Eisenoxid, umber-farbigem Eisenoxid, gelbem Eisenoxid und Gemischen hievon bestehenden Gruppe ausgewählt sind.

4. Zusammensetzung nach einem der Ansprüche 1 bis 3, wobei die genannte N-Acylaminosäure oder das Salz hievon von der aus N-Acyl-N-methylglycin, N-Acyl-N-methylalanin, N-Acyl-N-methylglutaminsäure und Gemischen hievon bestehenden Gruppe ausgewählt sind, und sich die genannte Acylgruppe von einer geradkettigen oder verzweigten C₁₀₋₅₀-Fettsäure herleitet, wobei sich die genannte Acylgruppe vorzugsweise von einer Fettsäure herleitet, welche von der aus Caprinsäure, Laurinsäure, Myristinsäure, Palmitinsäure, Stearinsäure, Ölsäure und Gemischen hievon bestehenden Gruppe ausgewählt ist.

5. Zusammensetzung nach Anspruch 4, wobei die genannten Salze der genannten N-Acylaminosäuren von der aus Aluminium-, Magnesium-, Calcium-, Zink-, Zirkonium-, Titansalzen und Gemischen hievon bestehenden Gruppe ausgewählt sind; wobei das genannte Salz vorzugsweise ein Aluminiumsalz ist.

6. Zusammensetzung nach Anspruch 5, worin das genannte Carbonsäurepolymer als Verdickungsmittel 0,10 % bis 0,50 % darstellt und von der aus Carbomeren, Acrylaten/C₁₀-C₃₀-Alkylacrylat-Kreuzpolymeren und Gemischen hievon bestehenden Gruppe ausgewählt ist; worin das genannte chemische Sonnenschutzmittel 1 % bis 10 % umfaßt und von der aus Ethylhexyl-p-methoxycinnamat, Octocrylen, Octylsalicylat, Oxybenzon, 2-Phenylbenzimidazol-5-sulfonsäure, 4,4'-Methoxy-t-butyldibenzoylmethan, 3- (4-Methylbenzyliden)kampfer und Gemischen hievon bestehenden Gruppe ausgewählt ist; und das genannte Feuchthaltemittel 1 % bis 5 % der genannten Zusammensetzung darstellt und von der aus Glycerin, Butylenglycol, Hexylenglycol und Gemischen hievon bestehenden Gruppe ausgewählt ist; worin das genannte Feuchthaltemittel vorzugsweise Glycerin ist.

7. Zusammensetzung nach Anspruch 6, wobei die genannte Zusammensetzung ferner 0,1 % bis 1 % von einem zusätzlichen Verdickungsmittel umfaßt, welches von der Gruppe ausgewählt ist, die aus Xanthangummi, Carboxymethylzellulose, Hydroxymethylzellulose, Hydroxyethylzellulose, Magnesiumaluminiumsilicat und Gemischen hievon besteht.

8. Zusammensetzung nach Anspruch 7, wobei die genannte Zusammensetzung ferner 1 % bis 4 % an Dihydroxyaceton umfaßt.

9. Verwendung einer Zusammensetzung zur Herstellung eines Arzneimittels zur Gewährleistung von Farbe, Befeuchtung und Schutz gegen die schädlichen Wirkungen von UV-Strahlung, welche Zusammensetzung umfaßt:
(a) 0,1 % bis 20 % von fein zerteilten Pigmentteilchen, welche mit einem unter N-Acylaminosäuren und Salzen hievon und Gemischen hievon ausgewählten Material oberflächenbehandelt wurden, um die Teilchen hydrophober zu machen,
(b) 0,025 % bis 1 % von einem Carbonsäurepolymer als Verdickungsmittel,
(c) 0,1 % bis 30 % von einem chemischen Sonnenschutzmittel,
(d) 0,1 Gew.-% bis 20 Gew.-% von einem Feuchthaltemittel und
(e) einen kosmetisch annehmbaren Träger.

## Revendications

1. Composition teintée en émulsion huile dans l'eau, utile pour procurer une couleur, une hydratation et une protection contre les effets néfastes du rayonnement UV, comprenant:
(a) de 0,1% à 20% de particules de pigment finement divisées ayant été traitées en surface pour rendre les particules plus hydrophobes avec une matière choisie parmi les N-acylaminoacides, leurs sels et leurs mélanges,
(b) de 0,025% à 1% d'un épaississant polymère d'acide carboxylique,
(c) de 0,1% à 30% d'un agent écran solaire chimique,
(d) de 0,1% à 20% en poids d'un humidifiant, et
(e) un véhicule acceptable en cosmétique.

2. Composition selon la revendication 1, dans laquelle lesdites particules de pigment constituent de 5% à 10% de ladite composition et lesdites particules de pigment sont choisies dans le groupe constitué par l'oxyde de fer, le dioxyde de titane, l'oxyde de zinc, l'oxychlorure de bismuth, le silicate de calcium, l'oxyde de chrome, l'hydroxyde de chrome, le ferrocyanure d'ammonium ferrique, le ferrocyanure ferrique, le kaolin, le violet de manganèse, le mica, la silice, le talc, l'outremer, et leurs mélanges; de préférence dans laquelle lesdites particules de pigment sont choisies dans le groupe, constitué par l'oxyde de fer, le talc, le dioxyde de titane, et leurs mélanges.

3. Composition selon la revendication 2, dans laquelle lesdites particules de pigment de type oxyde de fer sont choisies dans le groupe constitué par l'oxyde de fer noir, l'oxyde de fer brun, l'oxyde de fer orange, l'oxyde de fer rouge, l'oxyde de fer rouille, l'oxyde de fer terre d'ombre, l'oxyde de fer jaune, et leurs mélanges.

4. Composition selon l'une des revendications 1 à 3, dans laquelle ledit N-acylaminoacide ou son sel est choisi dans le groupe constitué par la N-acyl-N-méthylglycine, la N-acyl-N-méthylalanine, l'acide N-acyl-N-méthylglutamique, et leurs mélanges, et ledit groupe acyle est dérivé d'un acide gras en C₁₀-C₅₀ à chaîne droite ou ramifiée, de préférence dans laquelle ledit groupe acyle est dérivé d'un acide gras choisi dans le groupe constitué par l'acide caprique, l'acide laurique, l'acide myristique, l'acide palmitique, l'acide stéarique, l'acide oléique, et leurs mélanges.

5. Composition selon la revendication 4, dans laquelle lesdits sels desdits N-acylaminoacides sont choisis dans le groupe constitué par l'aluminium, le magnésium, le calcium, le zinc, le zirconium, le titane et leurs mélanges; de préférence dans laquelle ledit sel est un sel d'aluminium.

6. Composition selon la revendication 5, dans laquelle ledit épaississant polymère d'acide carboxylique constitue de 0,10% à 0,50% et est choisi dans le groupe constitué par les carbomères, les polymères croisés acrylates/acrylate d'alkyle en C₁₀-C₃₀, et leurs mélanges; ledit agent écran solaire chimique constitue de 1% à 10% et est choisi dans le groupe constitué par le p-méthoxycinnamate d'éthylhexyle, l'octocrylène, le salicylate d'octyle, l'oxybenzone, l'acide 2-phénylbenzimidazole-5-sulfonique, le 4,4'-méthoxy-t-butyldibenzoylméthane, le 3-(4-méthylbenzylidène)camphre, et leurs mélanges; et ledit humidifiant constitue del 1% à 5% de ladite composition et est choisi dans le groupe constitué par la glycérine, le butylèneglycol, l'hexylèneglycol, et leurs mélanges; de préférence dans laquelle ledit humidifiant est la glycérine.

7. Composition selon la revendication 6, dans laquelle ladite composition comprend, en outre, de 0,1% à 1% d'un épaississant supplémentaire choisi dans le groupe constitué par la gomme xanthane, la carboxyméthylcellulose, l'hydroxyméthylcellulose, l'hydroxyéthylcellulose, l'aluminosilicate de magnésium, et leurs mélanges.

8. Composition selon la revendication 7, dans laquelle ladite composition comprend, en outre, de 1% à 4% de dihydroxyacétone.

9. Utilisation d'une composition dans la fabrication d'un médicament pour procurer une couleur, une hydratation et une protection contre les effets néfastes du rayonnement UV, ladite composition comprenant:
(a) de 0,1% à 20% de particules de pigment finement divisées ayant été traitées en surface pour rendre les particules plus hydrophobes avec une matière choisie parmi les N-acylaminoacides, leurs sels et leurs mélanges,
(b) de 0,025% à 1% d'un épaississant polymère d'acide carboxylique,
(c) de 0,1 % à 30% d'un agent écran solaire chimique,
(d) de 0,1% à 20% en poids d'un humidifiant, et
(e) un véhicule acceptable en cosmétique.
